# EUROPEAN PATENT APPLICATION

(11) **EP 1 348 325 A2**
(43) Date of publication of application: **01.10.2003**
(21) Application number: 03013390.4
(22) Date of filing: 19.11.1998
(51) Int. Cl.: A01C 1/04, A01C 1/06, A01C 1/00, A01C 1/08

(54) **Solid matrix conditioning of seeds**

(30) Priority: 26.11.1997 US 979074
(62) Divisional of application: 98958091.5
(71) Applicant: Kamterter II, L.L.C., Lincoln, NE 68503 (US)
(72) Inventor: Eastin, John Alvin, Lincoln, Nebraska 68503 (US)
(74) Representative: UEXKÜLL & STOLBERG

(57) **Abstract**

The invention relates to a method of introducing characteristics into seeds during seed conditioning comprising the steps of admixing with continuous aeration of seeds, a particulate solid matrix material and a seed priming amount of water, for a time and at a temperature sufficient to imbibe sufficient water to enhance resultant plant vigor but insufficient to cause seed sprouting by bringing a substantial number of cells of the seeds to a desired stage of a cell cycle and adding genes to transform the seeds.

## Description

This invention relates to the inducing of characteristics into seeds as part of the conditioning of the seeds.

It is known from United States Patent 5,628,144, to prime seeds by mixing the seeds, a particulate solid matrix material and a seed conditioning amount of water, for a time and at a temperature sufficient to cause the seeds to imbibe sufficient water to bring the absorbing characteristics of seed material with the seed hull to the point of sprouting and then to dry the matrix for late planting. It is also known from "Systemic Acquired Resistance", by Scott Uknes, et al., Hort. Science, V. 30 (5), August, 1995, pages 962 and 963, to induce systemic resistance to pathogens and it is known from "In vivo and in vitro gene transfer to mammalian somatic cells by particle bombardment", by N.S. Yang, et al., Proc. Natl. Acad. Sci USA 87, 1990, pages 9568-9572, "Gene gun and other non viral approaches for cancer gene therapy", by N.S. Yang, et al., Nat.Med., Vol. 1, 1995, pages 481-483, and "Optimization of delivery of foreign DNA into higher-plant chloroplasts", by G.N. Yo, et al., Plant Mol. Biol., Vol. 15, 1990, pages 809-819, to introduce DNA or transfect plant cells with DNA. These methods are not as efficient as desired.

Accordingly, it is the purpose of the invention to provide a novel method of inducing characteristics into seeds during conditioning.

This purpose is accomplished by admixing with continuous aeration of seeds, a particulate solid matrix material and a seed priming amount of water, for a time and at a temperature sufficient to enhance resultant plant vigor but insufficient to cause seed sprouting characterized in that the seeds are brought to a state in which a substantial number of cells of the seeds are at a desired stage of a cell cycle and genes are added to transform the seeds. The transformed seeds may be planted. Advantageously, the transformed seeds may be primed before being planted.

Preferably, the seeds are continued in the matrix after transformation until the seeds are at the threshold of germination and than the matrix is dried and the primed seeds are planted under conditions not within one of a threshold germination temperature range and a threshold germination moisture range for unprimed seeds of the same species but within at least one of a threshold germination temperature range and a threshold germination moisture range for the primed seed. The matrix material may be removed before transformation or the matrix material may be planted with the seed. The genes may be introduced with a gene gun. Preferably the desired stage for introduction of DNA is one of the last portion of the S stage or the G2 stage.

The purpose is also accomplished to some extent by admixing with continuous aeration of seeds, a particulate solid matrix material and a seed priming amount of water, for a time and at a temperature sufficient to cause the seeds to imbibe sufficient water to enhance resultant plant vigor but insufficient to cause seed sprouting and adding a pathogen capable of inducing systemic resistance. The pathogen is included for a sufficient time to induce systemic resistance to the pathogen and full priming of the seeds followed by drying the matrix. The primed seeds are planted under conditions not within one of a threshold germination temperature range and a threshold germination moisture range for the unprimed seeds of the same species but within at least one of a threshold germination temperature range and a threshold germination moisture range for the primed seed. The matrix material may be removed before planting or may be planted with the seed.

The above noted and other features of the invention will be better understood from the following detailed description when considered with reference to the accompanying drawings in which:
FIG. 1 is a block diagram of a priming system in accordance with an embodiment of the invention;
FIG. 2 is a diagrammatic view of a portion of a mixing system used in the embodiment of FIG. 1;
FIG. 3 is a diagrammatic view of another embodiment of mixing system used in accordance with the embodiment of FIG. 1 instead of using the embodiment of FIG. 2;
FIG. 4 is a diagrammatic view of a conditioning system used in an embodiment of a portion of FIG. 1;
FIG. 5 is a diagrammatic view of a drying system used in accordance with the embodiment of FIG. 1;
FIG. 6 is a diagrammatic view of another embodiment of drying system in accordance with the invention; and
FIG. 7 is a diagrammatic view of still another embodiment of drying system used with the invention.

To prime seeds, the seeds are incorporated in a solid phase matrix comprising finely divided nonpathogenic, water-holding solids. Preferably, the matrix material, not including the seeds, is in such proportion and distribution with respect to the seeds so that the seed surfaces are sufficiently aerobic to favor aerobic metabolism of the seeds and of beneficial microorganisms, to deter the growth of unfavorable faculative anaerobes or anaerobic microorganisms and to permit proper metabolic changes. In most cases, this is accomplished by using the proper volumetric ratio of seed to matrix material and aeration.

The volumetric ratio of seed to matrix material should be for practical reasons in the range of one volume of seed to 120 of matrix material at one extreme to one of matrix to ten of seed at the other extreme. Preferably, a one to one ratio of seed to matrix is generally favorable but the ratio may be lower as in cases where seed exudation and germination inhibitor removal are significant seed priming and treatment objectives and higher such as when microbial inoculation calls for retaining exudates. In cases where the seed to matrix mixture contains a substantially larger volumetric ratio of seed over matrix, proper aeration during priming can be maintained through a combination of mechanical mixing and aeration of the seed plus matrix mass or more aeration.

The matrix material, when containing the water necessary to prime the seeds in question, should be sufficiently friable, nonclumping etc. so that, when desired, it can be separated from the treated seeds after treatment without damage to the seeds. The particle size of the matrix material is not unduly critical as long as surface area is adequate, but for mechanical reasons should be smaller or larger than the seed being treated, usually less than 20 mesh and preferably substantially different. Typically, a material less than about 60 mesh is preferred; for example the Agro-Lig described hereinafter was 90%/wt less than 200 mesh, the soft coal was less than 60 mesh.

One type of matrix is formed of organic solids, for example a carbonaceous, preferably a lignateous solid which has a large equilibrium water potential and preferably has an osmotic potential component which is at least about 90% and preferably greater than 95% of the total water potential, measured as described below. Examples of such material include coal, especially soft coal, lignateous shales such as the leonardite shale, sold as Agro-Lig, and sphagnum moss.

Another type of matrix material is inorganic such as calcined clay mineral, vermiculite and perlite. To achieve a practical cost-effective system using an inorganic particulate matter as the matrix, the bulk density of a matrix of that inorganic particulate matter is preferably above 0.3 grams per cubic centimeter. In this specification, bulk density means the weight in grams of a given volume of particulate material divided by its volumes in cubic centimeters. When using inorganic materials, the water potential is generally determined not primarily by osmotic potential but primarily by matric potential.

In the process of the invention, the seed to be treated, a predetermined amount of solid matrix material and a predetermined amount of water are admixed and the mixture allowed to equilibrate, preferably in a container designed to provide aeration but which reduces evaporative losses, for example, a closed metal container or bag, for a time and at a temperature sufficient to allow the seeds to imbibe water from the matrix and maintain or change a prescribed water content equilibrium temperature and aeration sufficient to enhance resultant plant vigor, i.e., enhance emergence, growth or yield characteristics, but short of that which would cause the seed to sprout. Particularly useful materials for the matrix are coal related materials, calcined clay, diatomaceous earth vermiculite, sawdust, perlite, peat/moss, corn cobs and grain dust.

As in solution priming, the equilibrium amount of water for the system to prime the seed is dependent on the specific seed variety, its state or condition, and the water potential of the solid matrix material. Typically the solid matrix material should have a water potential between about -0.5 to about -0.2 megapascals at equilibrium with the seeds. With control of temperature, this range may be extended to -0.2 to about -3.6. The exact conditions depend on the objectives and species such as destruction of inhibitors or physiological or physical blockages. The seed priming art to some extent is still empirical, and while typical water amounts and media water potentials for given seed types are already generally known from the solution priming art and solid state matrix priming experiments for some seeds, it is frequently best to test a small sample of a new seed over a readily determined range of water potentials and temperatures to determine what conditions of temperature, water potential and time cause appropriate imbibing of water by the seed and resultant pregermination events. After this priming, the seeds may be dried to a resting or dry storage state, with or without the matrix material. Treatment with beneficial microbes or chemical treatment may be before, during or after priming.

In one process of the invention, a known weight of seed is wet with about 25% by weight of water. The total water utilized is typically in the order of about one liter per kilogram of seed but varies with seed size and seed condition. The seed is mixed with the dry, flowable, particulate solid matrix material and water with appropriate chemicals or biologicals added so as to wet seeds and particulate matrix material uniformly. After the predetermined amount of water for priming is admixed with the coated seeds, the mixture is held at a predetermined temperature for a time sufficient to allow the seeds to maintain a desired moisture content equilibrium, usually one to about fourteen days. In other processes, the water, seed and matrix material are mixed in a continuous flow or batching blending system. The ratio of water may also vary substantially from 25 percent of the seed weight.

The seeds that can be treated can be virtually any seed, including most vegetable crops, ornamentals and agronomic crops. Included are cucumber, lettuce, carrot, onion, melons, sweet corn, tomatoes, eggplant, peppers, bean radish, squash, pea, flower seeds, alfalfa and soybean. Several different apparatus can be used for these procedures. One such apparatus is described below.

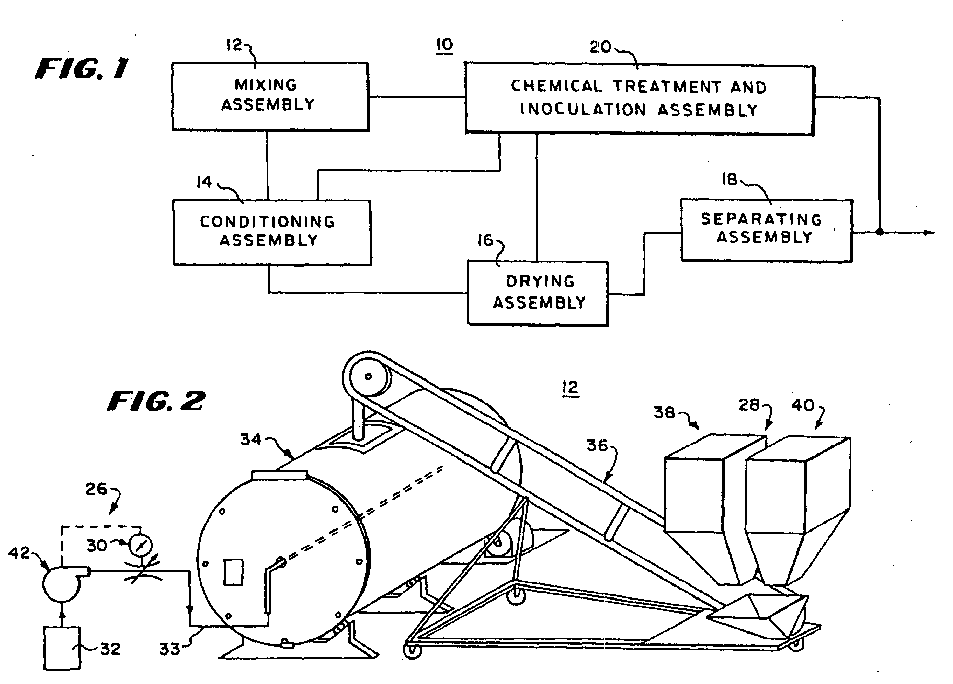

In FIG. 1, there is shown a block diagram of a priming system 10 having a mixing assembly 12, a conditioning assembly 14, a drying assembly 16, a separating assembly 18 and a chemical treatment and inoculation assembly 20. In this system, seeds and a matrix are: (1) mixed together either with no chemical treatment or inoculation of the seeds or with chemical treatment and/or inoculation of the seeds; (2) conditioned; (3) dried back; and (4) in some embodiments, the matrix medium and seeds are separated.

The chemical treatment or inoculation generally takes place in the chemical treatment and inoculation assembly 20 which may communicate with the mixing assembly 12, the conditioning assembly 14, the drying assembly 26 or the separating assembly 18 or none of them. The mixed matrix, seeds and water are utilized for pre-germination metabolism, after which the seeds may be dried back in the drying assembly 16 although from time to time the drying assembly 16 and and conditioning assembly 14 are used intermittently for staged conditioning and drying. The drying assembly 16 may communicate with the separating assembly 18 to separate the matrix from the seed in some embodiments although such separation is not always required. The treated seeds may be stored and used later or immediately utilized.

Not all seeds are subject to chemical treatment or inoculation, and thus, the chemical treatment and inoculation assembly 20 is not always used. However, in some embodiments, the seeds may be chemically treated or inoculated with microbes prior to being mixed in the mixing assembly 12 with water and matrix, or in the alternative, such treatment may be applied in the conditioning assembly 14, the drying assembly 16 or later after preconditioning.

In some embodiments, the beginning stages of metabolism occur until the beginning of germination as indicated by the emergence from the seed hull of embryo, at which time inoculation with beneficial microbes takes place. After the inoculation, the seeds may be returned to the conditioning assembly 14 for healing to be restored to a pre-germination stage without full germination and be dried back for later use.

In FIG. 2, there is shown a schematic view of a mixing assembly 12 having as its principal parts a water source 26, a solid matter source 28 and a rotatable mixing tank 34 connected together so that the source of water or water plus additives such as potassium nitrate and the solid matter source 28 such as matrix material and seeds communicate with a rotatable mixing tank 34 which combines the ingredients into the proper mixture for oxidation, temperature control and water potential control. All of these sources may pre-mix the solid matter and moisture and then apply it to the mixing tank or they may be individually applied or combined to pairs and applied or combined with treatment material or the chemical treatment material may be applied to any of the ingredients such as the source of water or the source of matrix or to the seeds alone.

The source of water 26 includes a meter 30, a source of water 32, and in some embodiments a pump 42 connected together so that a metered amount of moisture may be applied to a mixing mechanism, which in the embodiment of FIG. 2, is a rotatable tank. It is adapted to receive the water and solid matter and then to be rotated for thorough mixing. Of course, other embodiments may be utilized such as a tank with internal stirring means rather than a rotating tank and/or connections with rotatable universal joints that rotate with the rank rather than connectors at the center of rotation.

The solid matter source 28 includes a seed and solid matrix conveyor 36, a source of seeds 38, and a source of matrix material such as clay 40. These ingredients may be premixed or moved one-by-one into the tank 34 by an auger or forced air flow or gravity or any other means.

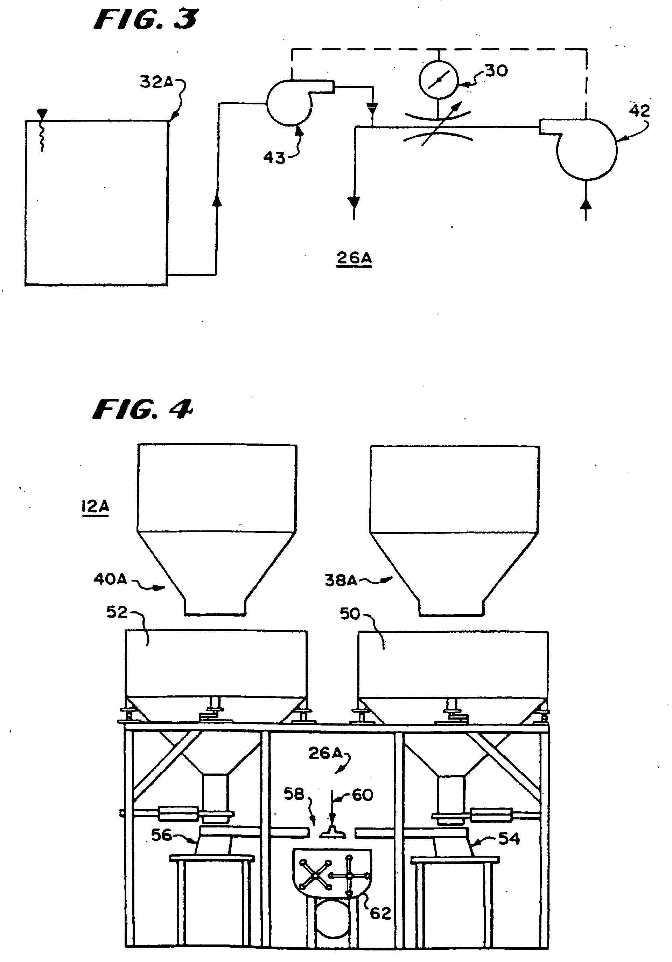

In FIG. 3, there is shown an embodiment of water source 26A having a tank of water 32A which contains water and potassium nitrate. The tank communicates with a pump 42 and a meter 30 in a conduit 31 for supplying a mixture of liquid ingredients which may include additives such as potassium nitrate to condition for light sensitivity or chemicals beneficial to seed treatment or being antifungal or antimicrobial or with beneficial microbials for inoculation and in some embodiments, nutrients therefore.

With this arrangement, a carefully metered amount of water may be supplied to provide the correct matrix potential. In some applications, a small amount of water is supplied and then the supply is disconnected from the tank which is rotated for mixing and then further water is supplied. The intermittent stationary portions of the tank may be utilized to also add more dry matter if desirable so that the mixing together may proceed with stepped amounts of dry matter and water.

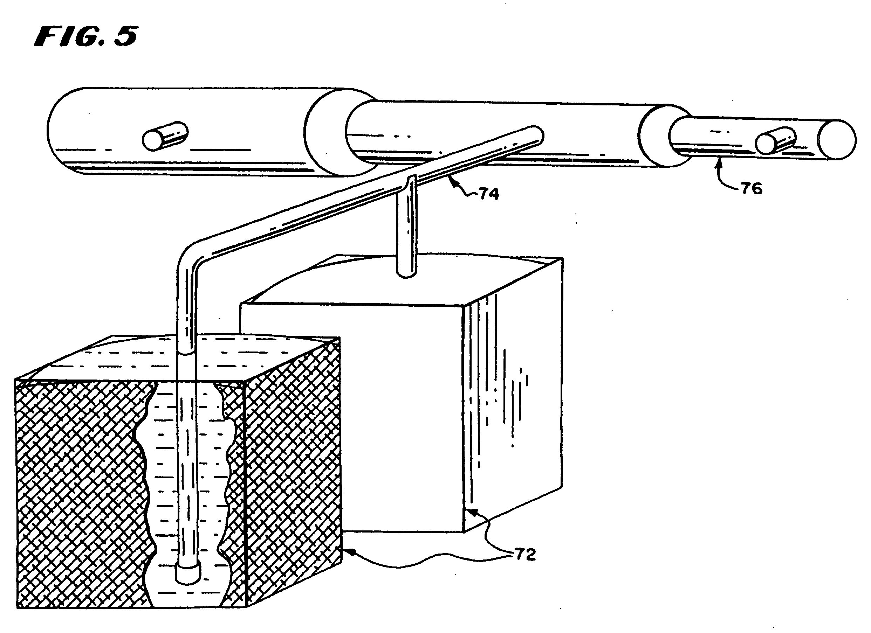

In FIG. 4, there is shown a schematic diagram of another embodiment of mixing assembly 12A having a source of seeds 38A, a source of dry matter 40A, the source of water and liquid additives 26A, a mixing section 58, and a mixture agitator and transporter 62. The source of seeds 38A and the source of dry matter 40A each include a different one of the hoppers 50 and 52 respectively for containing bulk seed and dry matter such as matrix. At the bottom end of each, there is a corresponding one of the seed conveyors 54 and conveyor 56 which are aligned directly in the line of gravity underneath the seed and matrix hoppers 50 and 52 respectively and are drivable at controlled speeds independently to transport from the hoppers controlled amounts of material for mixing in the mixing station 58.

The bottoms of the hoppers are adjustable and the speed of the containers are adjustable so that the ratio of mixing may be proportional in accordance with the type of seed and the type of matrix material. Similarly, the water supply 26A supplies water at a controlled rate through a conduit 60 into the mixing station 58 so that controlled amounts of matrix material, seed and water or water solutions are mixed together at the mixing station 58 in a continuous process for thorough mixing with the station 58 receiving at any one time between 1 percent and 60 percent of the total amount a batch to be processed during batch processing, or if continuous processing is used, a volume of properly proportioned materials which is adapted to fit into a flow stream no faster than the slowest processing of that stream. The proportional mixtures are mixed at the station 58 and conveyed by the mixer, agitator and transporting system 62 which moves the mixture along by a series of panels to an outlet where it may be conveyed to the seed conditioner. The seed conditioner may accumulate it batch by batch for processing or the seeds may be conditioned in a continuous process.

In FIG. 5, there is shown a conditioner 14 which includes a means for controlling the temperature, moisture and oxygen supply of the matrix containing seeds to promote pre-germination conditioning. In the embodiment of FIG. 5, all of these functions are performed in a unitary manner by a porous fabric bag 72 which contains the matrix and seeds, a suction manifold 74 positioned for even moisture and temperature drawing of air through the fabric bag 72 and matrix to a vacuum manifold 76 and a temperature and moisture controlled room for this processing. For very small amounts, the air drawing supply 74 is unnecessary since there will be adequate natural transfer of heat and oxygen through the matrix material for the desired purposes.

The fabric bag 72 has a open end for supplying the mixture to the bag and an openable bottom end for supplying conditioned seeds to a transport conveyor 80 after a conditioning stage. In practice, the seeds may be conditioned in the fabric bag 72 and then transported for drying back through a 10 percent stage of drying and then returned to the bag for further conditioning. Moreover, the conditioning may extend until the beginning of emergence and inoculated and dried and conditioned again prior to emergence.

To draw air through the fabric bag 72, a centrally located vacuum pipe 74 extends downwardly. It is designed so that the larger surface area on the outside of the fabric bag 72 pulls cool dry air through the wider area, and as it picks up some heat and moisture, the rate of flow becomes greater since it is moving inwardly through spheres of smaller area to maintain uniformity of temperature through the bag and relative uniformity removal of moisture. In the preferred embodiment, the bags generally contain between 20 pounds and 1,000 pounds of mixture, contained in a room that is between 10 degrees Centigrade and 15 degrees Centigrade with air being pulled from a manifold under a vacuum pressure of a negative 10 pascals through an outer surface area of 75 square feet to an inner perforated conduit having a diameter of between 1 inch and 15 inches and open pores and ends for drawing air having a combined area of 75 square centimeters.

After a conditioning stage, the fabric bags 72 may be physically moved to driers and dumped into the driers in a manner shown herein or the bottoms may be opened and conveyor mechanisms 80 may convey the mixture to driers or to a drying stage.

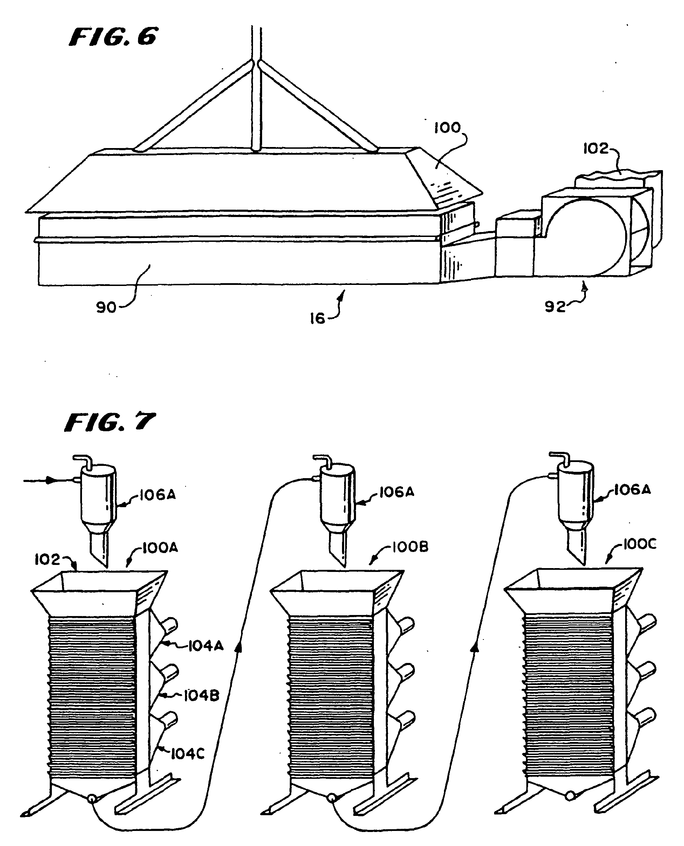

In FIG. 6, there is shown one embodiment of drying assembly 16 having a bin assembly 90, a blower assembly 92, and in some embodiments a cover assembly 100. A source of air pressure 102 applies air through blowers or the like to the blower assembly beneath the bin assembly 90. The bin assembly 90 has a fabric bottom through which the air pressure blows to cause turbulence in the matrix material spread across the bin assembly 90 for drying thereof by air flow. A cover may be placed over the bin assembly 90 to create slight negative pressure if desired for dust control and moisture may be extracted by recirculation of the air through desiccators in the cover.

In one embodiment, air pressure in the manifold beneath the bin assembly 90 of a positive 50 pounds per square inch is applied through a 60 gauge fabric for drying matrix material applied in a layer between 4 inches and 3 feet deep and having a pressure on the fabric of between 6 ounces and 15 pounds per square inch.

In FIG. 7, there is shown another embodiment of drying assembly having a plurality of drying towers 100A, 100B, and 100C each of which receives a mixture of matrix and seed for drying with the seed being applied to the top of the tower 100A through a conveyor 106A which may be an auger or an air blower or the like for filling the tower 100A. The tower 100A is emptied into the tower 100B through a similar conveyor 106A and so on down the line until the drying operation is completed, after which the mixture may be moved to a separator for sifting and separating or may be stored for use in a planter with the matrix material and seed combined or moved to a location for chemical treatment or biological inoculation. Each of the towers is identical and so only the tower 100C will be described.

To provide a stage of drying, the drying tower 100C contains an elevated bin 102 having a plurality of stages of air flow conduits 104A, 104B and 104C. Any number of such stages may be supplied and they include within them a blower for blowing air crosswise through the tower where it is received by the next section for blowing crosswise again through the tower from the opposite side forwardly so that the air blows across the tower through several stages, three being shown for illustration in FIG. 7. A desiccant or other dryer may be utilized at each stage to remove moisture from the air as it dries the mixing combination.

The solid matrix priming compositions described above can also advantageously contain: (1) prescribed amounts of known seed fungicides such as thiram, captan, metalaxyl, pentachloronitrobenzene, and fenaminosulf, so that the finished primed seed is coated with or absorbs the desired amount of fungicide, such as is known to those skilled in the art; (2) microorganisms useful to the crop such as those useful in crop protection, stimulation or establishment, and for such purposes, some significant bacteria are strains of: Bacillus enterobacter, Pseudomonas, Rhizobia and Serratia species and some significant fungi are strains of Trichoderma, Gliocladium and Laetisaria species; (3) pesticides such as fungicides or bactericides included prior to, during or after the solid matrix priming; and (4) growth regulators such as potassium nitrate, gibberellic acid, DCPTA, ethephon.

In this specification, "solid matrix priming" is considered the process whereby seeds are intimately mixed with particulate solid phase media in the presence of sufficient water to realize a moisture content in the seed which allows for germination processes to occur but which prevents radicle emergence. During priming and especially during solid state priming, the addition of chemical additives and microorganisms is especially beneficial. In this specification, the language "added beneficial microorganisms" means a beneficial microorganism that is added to the composition at a level in excess of that naturally occurring on the seed being primed.

In this specification: (1) threshold germination temperature range means that range of temperatures for a certain species within which seeds of that species will germinate at a predetermined moisture level and with adequate oxygen; and (2) threshold germination moisture range means that range of moistures for a certain species within which seeds of the species will germinate at a given temperature and with adequate oxygen.

In addition to the above ranges, the limiting points of moisture and temperature in this specification are defined as follows: (1) threshold germination temperature base means that temperature below which a seed for a given species will not germinate even though the oxygen and moisture levels are acceptable; (2) threshold germination critical maximum temperature means that temperature above which a seed of a given species will not germinate even though there is an acceptable moisture level and adequate oxygen; (3) threshold germination moisture base means that moisture level below which a seed of a given species will not germinate even though the temperature and oxygen are appropriate; and (4) threshold germination moisture critical maximum level means that moisture level in soil above which a seed of a given species will not germinate even though the temperature and oxygen are adequate.

Other convenient terms are defined as follows: (1) emergence rate index means the sum of the emerged seeds, n, on each day multiplied by a quantity for that day, quantity for that day being equal to the total number, c, of days in the assay minus the count, n, of seedlings on that day; and (2) synchronization factor means the emergence rate index that occurs in the period that is one-quarter of the time into the total assay period. The assay period is a normal field crop emergence period. The emergence rate index and synchronization factor can be calculated using seeds germinated instead of seedlings emerged to arrive at a similar number. This may be called a germination rate index.

The above values are determined empirically for a given seed and variety and may be used in planting.

A sufficient amount of matrix or a matrix of material sufficiently adsorbent or absorbent to remove enough inhibitors to prevent delay of more than fifteen percent of the time duration of activation prior to a control time of germination in which the inhibitor is substantially completely removed by an appropriate washing technique is required. The time of germination in this specification is that the visible radicle protrusion can be observed and the activation period occurs when membranes become differentially permeable and conversion occurs from substantially passive solute transport in and from the seed. The amount and type of matrix is also selected to either retain or permanently remove exudates so that: (1) in the case of removal, it is not deleterious such as for example, to encourage pathogen growth; or (2) in the case of retention, to enable growth of innoculated beneficial microbes on seeds.

The matrix priming process also provides a superior approach to SAR (Systemic Acquired Resistance) as described in "Systemic Acquired Resistance" by Scott Uknes, et al., in Hort Science, Vol. 30(5), August, 1995, pages 962 and 963 and other publications. For example, using this process, additives may be mixed with the seeds during conditioning described above to induce immunity. Both pathogens and non-pathogens are able to induce immunity. Moreover, the known seed funigicides described above such as thiram, captan, metalaxyl, pentachloronitrobenzene, fenaminosulf and some growth regulators including those mentioned above may have this effect. Systemic acquired resistance is aided by the conditioning described herein, particularly when the matrix includes lignin. The priming process stimulates certain genes that cause resistance to pathogrens. This is sometimes referred to in the literature as a "signal" that permits the induced immunity to some pathogens that are present at the time of the signal.

The seed conditioning method and apparatus described herein may be used efficiently for other transgenetic processes besides the spraying with inoculum. It may be used efficiently because it provides a technique for placing cells in an efficient condition for the introduction of genes. Generally, the seeds may be placed into the last part of the S stage or the G-2 stage of the cell cycle, at which stage, genes may be integrated into the separated DNA which is in ample supply in large numbers of the seeds that have been primed or conditioned into the G-2 stage.

The time, temperature and moisture environment necessary to bring the seeds to this portion of the cell cycle can be determined during the conditioning process described herein before as a portion of the priming operation. During the absorption of water controlled by water potential in the priming media, the seeds may be sampled to determine the conditions that bring them to the desired cell cycle, such as for example, the G-2 portion of the cell cycle or the latter part of the S phase. With this information, the seeds may be brought into that portion of the cell cycle using production techniques and then vectors introduced such as by gene guns or any other device that will introduce genes for integration into the DNA of the cells.

It is not fully understood why synergistic results are achieved from the use of added microbes and chemicals during priming but such results have been shown by tests. The data demonstrates synergism with regard to solid matrix priming and the utility of the technique in eliciting beneficial stand establishment effects when used with beneficial microorganisms in addition to the effect of either treatment alone. Other tests show that the synergism when added to the solid matrix priming system as well.

The inoculation of seeds during matrix priming may be used as an inoculation system to introduce beneficial microbes into soil by inoculating seeds with beneficial microbes and permitting them to grow. In this specification, the words "beneficial microorganisms" or "beneficial microbes" means microorganisms inoculated by seeds and which are any of those many microbes that: (1) have been identified in association with plant roots, soils and seeds and/or as endophytes and determined to result in growth substance mediated plant growth promotion, improved plant nutrition and pest control through biocontrol and/or improved plant health to have potential utility in agriculture; (2) have been identified or can be further selected or engineered from those already identified to degrade specific chemical agents in soils to have potential utility in environment bioremediation if effectively delivered to the target site; and (3) are believed to be useful in supporting and protecting somatic embryos (synthetic seeds) as a substitute for zygotic seeds to deliver genetic characteristics of species which cannot be handled as zygotic seeds or to propagate elite germplasm and hybrids that are not now possible. The use of somatic embryos is described in Cantliffe DJ, Liu JR (1987), "Development of artificial seed of Sweet potato for clonal propagation through somatic embryogenesis in: Methane from biomass: A systems approach", Smith WH, Frank JR (eds) Elsevier, New York, pp 183-195.

Beneficial microbes in association with plants are ubiquitous in natural soil environments and serve useful functions in removing contaminants from the ecosystems. Such beneficial microbes are described in John Davison, "Plant Beneficial Bacteria", Bio/Technology, pages 282-284 and Chet, et al., "Mechanisms of biocontrol of soil-borne plant pathogens by Rhizobacterial", D.L. Keister and P.B. Cregan (Eds.), The rhizosphere and plant growth, 229-236. However, with the exception of Rhizobium and Bradyrhizobium genera, none of the many beneficial rhizosphere, soil or seed based microbes that have been identified has been extensively commercialized. The problem is not whether useful microbes can be identified but the economic delivery of a reproducibly useful inoculum at the target site.

The potential utility of beneficial microbes in managed terrestrial ecosystems for agriculture and environment bioremediation is substantially dependent on delivering adequate numbers of targeted beneficial microbes to the ecosystem site/niche where activity is needed at an economical cost.

However, it has been discovered that seed inoculation and the subsequent growth of the target microbe in the root environment provides an economical mechanism for delivering adequate numbers of beneficial microbes to a terrestrial environment. The bulk of the inoculum increase takes place using photosynthesis as energy and the rhizoplane, rhizosphere or plant endosphere as the bioreactor site.

Numerous genera of plant growth promoting rhizobacteria (PGPR) have been reported to have growth promotion and biocontrol activity (Schroth and Hancock, 1981). Their review was over a decade ago and many other reports have been made since that time. However, PGPR usually fail in the field because of poor bacteria distribution on roots and failure of their antagonistic activity toward detrimental rhizosphere microbes (Schippers, et. al., 1987). It is generally believed that a critical threshold population of a particular PGPR must be established to be effective and the method of application is critical (Weller, 1988). The solid matrix seed priming (SMP) process and a solid matrix seed priming based microbial inoculation process (SMPI) which has been effective in establishing large PGPR numbers on seeds and has resulted in effective seedling establishment in disease stress situations as shown in Table 10 (Table 1, Eastin, 1987, Solid matrix priming of seeds with microorganisms and selected chemical treatment, U.S. Patent Application No. 07/440,470; Eastin and Harman, 1987, Biological control and solid matrix priming, U.S. Patent Application No. 034,813). The data in table 10 are representative of results with Pseudomonas flourescens. Pseudomonas putida, Enterobacter cloacae and Trichoderma strains that have been tested. It can be understood from this table that there is a large differential achieved if bacteria are applied during priming as contrasted to after priming. It is believed the superior emergence response from SMPI under disease pressure as shown in Table 10 is a function of bacterial colonization of the seeds as contrasted to the lesser response with equivalent numbers of the same bacteria applied topically.

The SMPI system is based on the concept that microbes function in nature as colonies and the SMPI process provides microorganisms the opportunity to grow and colonize on hydrated seed surfaces. Priming seeds and the process of growing microbes on seeds during priming synchronizes radicle emergence and seedling development better with microbial activity from colonies established on the seed. The large log increase in bacteria numbers in/on the seeds appears to mean that low inoculum levels can result in large seed bacteria population if the proper inoculation system is used. This has large economic implications as inoculum can be increased by growing on the seed per se.

To inoculate soil with a beneficial microbe of any of the types described above, seeds are primed by mixing with the seeds a particulate solid matrix material and a seed priming amount of water, for a time and at a temperature sufficient to cause the seeds to imbibe sufficient water to enhance resultant plant vigor but insufficient to cause seed sprouting wherein said mixture has a water potential between about -0.5 to about -2.0 megapascals at equilibrium with said seeds and said particulate solid matrix material is nonpathogenic to the seeds and to the beneficial microbes. The primed seeds are planted under conditions not within one of said threshold germination moisture range and threshold germination moisture range for the unprimed seeds of the same species but within said range for the primed seed, wherein the beneficial microorganism is grown on the seed and matrix during priming matrix material and is planted with the seed and the seeds and soil are innoculated with a beneficial microorganism. The priming process is selected to be suitable for the beneficial microorganism. Thus, after colonizing the seed and matrix, the beneficial microorganism is present in colony numbers on the seed and carried to the soil.

In some cases, harmful agents can be destroyed or rendered ineffective during priming by antibiosis. For example, Psuedomonas gladioli which is a fungistatic bacteria has been inoculated onto sweet corn seeds by introduction in moisture during solid matrix priming and killed during dry back and storage. It acted as a powerful fungicide against Pennicilium and Fusarium species, thus SMPI can serve as an effective seed treatment process as well as a vehicle for effective seed and soil beneficial microbe inoculation.

While a preferred embodiment of the invention has been described with some particularity, many modifications and variations in the preferred embodiment may be made without deviating from the invention. Accordingly, within the scope of the appended claims, the invention may be practiced other than as specifically described.

## Claims

1. A method of introducing characteristics into seeds during seed conditioning comprising the steps of admixing with continuous aeration of seeds, a particulate solid matrix material and a seed priming amount of water, for a time and at a temperature sufficient to cause the seeds to imbibe sufficient water to enhance resultant plant vigor but insufficient to cause seed sprouting **characterized by** bringing a substantial number of cells of the seeds to a desired stage of a cell cycle and adding genes to transform the seeds.

2. A method according to claim 1 **characterized in that** the transformed seeds are planted.

3. A method according to claim 1 **characterized in that** the transformed seeds are primed and planted.

4. A method according to any of claims 1-3 further **characterized by** continuing the seed in the matrix after transformation until the seeds are on the threshold of seed sprouting;
drying the matrix;
planting the primed seeds under conditions not within one of a threshold germination temperature range and a threshold germination moisture range for unprimed seeds of the same species but within at least one of a threshold germination temperature range and a threshold germination moisture range for the primed seed.

5. A method according to any of claims 1-4 **characterized in that** the matrix material is removed before transformation.

6. A method according to claim 5 **characterized in that** the matrix material is planted with the seed.

7. A method according to claim 6 **characterized in that** genes are introduced with a gene gun.

8. A method according to any of claims 1-7 **characterized in that** the desired stage is one of the last portion of the S stage or the G2 stage.

9. A method of introducing characteristics into seeds during seed conditioning comprising the step of admixing with continuous aeration of seeds, a particulate solid matrix material and a seed priming amount of water, for a time and at a temperature sufficient to cause the seeds to imbibe sufficient water to enhance resultant plant vigor but insufficient to cause seed sprouting **characterized by** adding a pathogen capable of inducing systemic resistance.

10. The method of claim 9 **characterized in that** the pathogen is included for a sufficient time to induce systemic resistance to the pathogen and full seed priming followed by drying the matrix and planting the primed seeds under conditions not within one of a threshold germination temperature range and a threshold germination moisture range for the unprimed seeds of the same species but within at least one of a threshold germination temperature range and a threshold germination moisture range for the primed seed.

11. A method according to claim 10 **characterized in that** the matrix material is removed before planting the seed.

12. A method according to claim 10 **characterized in that** the matrix material is planted with the seed.
